# EUROPEAN PATENT APPLICATION

(11) **EP 2 210 971 A1**
(43) Date of publication of application: **28.07.2010**
(21) Application number: 09425018.0
(22) Date of filing: 26.01.2009
(51) Int. Cl.: D04B 1/22, A61L 27/18

(54) **Silk fibroin textile structures as biomimetic prosthetics for the regeneration of tissues and ligaments**

(71) Applicant: Politecnico Di Milano, 20123 Milano (TN) (IT); Stazione Sperimentale per la Seta, 20133 Milano (IT)
(72) Inventor: Freddi, Giuliano, 20133 Milano (IT); Tanzi, Maria Cristina, 20133 Milano (IT); Fare' , Silvia, 20133 Milano (IT); Alessandrino, Antonio, 20133 Milano (IT); Calimani, Roberto, 20129 Milano (IT)
(74) Representative: Long, Giorgio

(57) **Abstract**

The invention concerns a silk fibroin weave structure comprising a concentric tubular outer sheath and an inner core, wherein said sheath is a woven fabric with interlaced loop and said core is a woven fabric with interlaced loop or is braided using a braiding machine.

The silk fibroin used to weave the structure of the invention is a silk yarn with a linear density comprised of between approx. 40 den and approx. 640 den, preferably, the linear density is approx. 80 den or approx. 320 den.

The core of the structure has a mean pore area comprised of between 0.001 and 2.000 mm², while the outer sheath has a mean pore area comprised of between 0.005 and 15.000 mm²_{.}

The structure of the invention is used for the in vivo or *in vitro* regeneration of ligaments, particularly the anterior cruciate ligament, tendons, muscles and blood vessels. Furthermore, the structure promotes *in vivo* and *in vitro* cell adhesion and proliferation.

## Description

### BACKGROUND OF THE INVENTION

The present invention relates to the technical field of silk fibroin biomimetic prosthetics for the regeneration of tissues, particularly ligaments and tendons.

Traumatic events are the major cause of lesions and ruptures of the knee joint ligaments, particularly, the anterior cruciate ligament (ACL) is the structure most commonly compromised.

Prosthetic reconstruction is the most commonly performed treatment following injury to ligaments or tendons, particularly in the case of younger patients.

Biological, synthetic and natural ACL substitutes have been adopted over the years. Currently, as a result of the poor long-term results, synthetic materials have been abandoned and the use of autologous grafts, *i.e*. tendon or ligament tissue taken from the patients themselves, has become increasingly common.

The silk fibroin textile structures described herein as "biomimetic prosthetics" for the regeneration of tissues and ligaments, are presented as alternative solutions to the use of autologous grafts for the regeneration of injured tissues, mainly tendons and ligaments, particularly the ACL.

The innovative concept of "biomimetic prosthetics" introduced herein envisages that, once implanted in the patient's body in place of the damaged tendon or ligament, this represents the structural support on which new ligament tissue is formed.

Therefore, a biomimetic prosthesis should be manufactured from a material capable of promoting cell adhesion and proliferation, without its subsequent degradation compromising cell metabolism and eliciting an abnormal inflammatory response from the host.

The mechanical characteristics of the biomimetic prosthesis must guarantee functional continuity between the implant and the surrounding tissues *in vivo*. Furthermore, the porosity and geometry of the pores must be such as to also allow cellular migration within the structure and promote the passage of oxygen, metabolites and growth factors.

Particularly, a biomimetic prosthesis for the regeneration of the anterior cruciate ligament must satisfy the following characteristics:
- mechanical properties comparable to those of the human ACL, which possesses a mean rupture load value of approx. 2000 N;
- the ability to allow cell colonisation and the formation of new tissue in all its parts;
- such a rate of degradation as to guarantee the gradual transfer of load onto the new ligament and ensure the mechanical stability of the joint until tissue regeneration is complete.

### STATE OF THE ART

Potential matrices for ACL regeneration investigated to date are produced starting from natural polymers (for example collagen and silk fibroin) and synthetic polymers (for example polylactic acid or polyglycolic acid and copolymers thereof, polyDTEcarbonate).

Over the years, silk fibroin has proven to be an ideal candidate due to its high mechanical properties, biocompatibility and its stability in a chemically aggressive environment, such as that of biological fluids, where low rates of degradation are observed.

Silk is a naturally derived protein, produced in the form of fibres by certain species of lepidoptera and arachnids, for the creation of support and protective structures. Depending on the species producing it, since the roles the fibres must play differ, the different types vary in terms of fibre composition, structure and mechanical properties. The type of silk most studied for biomedical applications is that produced by the caterpillars of *Bombyx mori.*

The silk from *Bombyx mori* is composed of two major proteins, sericin and fibroin. Fibroin, a fibrous protein, is produced as long strands covered with sericin, a globular protein, which acts as an adhesive, holding the fibroin strands together.

Silk can only be used in biomedical applications inside the human body if deprived of the sericin, which has been shown to provoke a strong inflammatory response by the body. Removal of the sericin from silk fibres is performed by means of a physico-chemical process known as degumming. The degumming process can be performed in various ways, such as high temperature treatment in an alkaline solution in the presence of detergents, or by treatment in aqueous solutions containing sodium carbonate or even by means of autoclave treatments in the presence of superheated water. Following purification of the fibres from sericin, no anomalous inflammatory reactions and hypersensitivity by the host body are observed.

The mechanical properties of fibroin include high toughness and strength. The combination of the microstructural characteristics of fibroin fibres gives superior mechanical properties compared to those of other fibres (Table 1) obtained both from naturally derived polymers (for example cotton and collagen, used in the textile and biomedical fields respectively) and synthetic polymers (for example Nylon fibres used in the textile sector or those obtained from polylactic acid, a material approved for biomedical applications).

**Table 1 - Comparison between the mechanical properties of Bombyx mori silk fibroin and other polymeric fibres**

| **Material** | **Elastic modulus (GPa)** | **Ultimate strain (MPa)** | **Elongation at rupture (%)** |
|---|---|---|---|
| Fibroin | 10 | 740 | 20 |
| Nylon | 3 | 500 | 18-26 |
| Cotton | **6 - 11** | 300-700 | 5-7 |
| Collagen | 0.0018-0.046 | 0.9-7.4 | 24-68 |
| PLA | 1.2-3.0 | 28-50 | 2-6 |

Once implanted inside the human body, fibroin undergoes proteolytic degradation. The rate of degradation of fibroin is defined by numerous factors depending on the implantation conditions and the shape, geometry and size of the structure under consideration. For example, it has been observed that fibroin suture threads, implanted *in vivo,* lose the majority of their tensile strength within one year and degrade completely within two years of implantation. The cytocompatibility of fibroin has been confirmed by various research groups, who have also shown the ability of fibroin to support cell adhesion and proliferation, to an extent comparable with collagen.

Fibroin offers the possibility of being processed in various forms such as foams, hydrogels, films and fibres, allowing the attainment of biomimetic prosthetics of versatile geometries, able to be used in the regeneration of various tissues and, particularly, seeming to possess all the necessary requirements for the creation of a fibrous matrix for the regeneration of ligaments and tendons.

In 2002, at Tufts University in Boston, Altman *et al.* developed a matrix for the regeneration of the anterior cruciate ligament, using fibroin fibres organised according to a complex hierarchical structure, with a geometry similar to that of a cord (US 6,902,932 and G.H. Altman, R. L. Horan, H. H. Lu: "Silk matrix for tissue engineered anterior cruciate ligaments"; Biomaterials, 2002, 23, 4131-4141).

Analysis of the experimental results has shown that said matrix possesses mechanical characteristics comparable to those of the natural ACL, but has such an excessively compact structure as to make cell colonisation within the matrix difficult.

From 2003 to date, in the Biomaterials Laboratory, Department of Bioengineering, Milan Polytechnic and the Silk Experimental Station, various types of fabric matrices have been made using yarn obtained from fibroin fibres according to different morphologies (A. Alessandrino: "Silk textile structures as scaffolds for the engineering of the anterior cruciate ligament"; Degree Thesis submitted to Milan Polytechnic, academic year 2003/2004; M. Colombo: "Silk scaffold for the reconstruction of the anterior cruciate ligament. Mechanical characterisation and *in vitro* biodegradation kinetics"; Degree Thesis submitted to Milan Polytechnic, academic year 2004/2005). Despite demonstrating the capacity to allow cell colonisation, even in the most internal layers, the matrices thus obtained have shown mechanical characteristics that are incompatible with those of tissues such as tendons and/or ligaments.

As a conclusion to the analysis of the state of the art conducted, it is possible to state that the silk fibroin matrices currently known to those skilled in the art have not been shown to possess the range of characteristics necessary to effectively promote the regeneration of tissues such as tendons and ligaments, particularly, the ACL.

### DESCRIPTION OF THE INVENTION

The ideal structure for ligament regeneration should show a good compromise between mechanical properties and porosity. This structure must be capable of supporting the load required of the ligament or tendon in the medium term and, progressively, self-degrade in such a way that the newly formed tissue integrates with the host tissue, replacing the structure itself. Furthermore, the structure must possess such porosity as to guarantee that cell colonisation occurs throughout its entire volume and not just on the outer surface.

The characteristics described herein are those initially indicated for the "biomimetic prosthesis", however, the technical drawback the present invention must answer is that of providing a silk fibroin biomimetic prosthesis with mechanical characteristics comparable to those of natural ligaments and tendons and, at the same time, have such morphology as to allow colonisation by cells and the development of novel tissue within its interior.

Said problem has been met and resolved by means of the development of a silk fibroin weave structure as depicted in the appended claims.

The present invention concerns a tubular silk fibroin structure obtained by particular weaving and webbing with such macroscopic porosity as to allow cell colonisation in every part, and at the same time, mechanical properties comparable to natural ligaments, particularly, the ACL.

Further characteristics and the advantages of the structure of the invention will emerge from the following description of its preferred embodiments, given for nonlimiting indication, with reference to the enclosed figures, wherein:
Figure 1 shows a sketch of a silk yarn with linear density equal to approx. 80 den made from approx. four twisted ply, each consisting of 10 silk filaments;
Figure 2 shows a sketch of a silk yarn of linear density of approx. 320 den formed by 4 twisted silk yarns of approx. 80 den;
Figure 3A shows a sketch of an overall core-sheath structure where both the,core and the sheath are made from knitted weave with interlaced loop using an 80 den raw silk yarn;
Figure 3B shows the same structure as figure 3A showing a detail of the internal core;
Figure 4A shows a sketch of a core-sheath structure wherein the sheath is made from knitted weave with interlaced loop while the core is made using a braiding machine with 80 den raw silk yarn;
Figure 4B shows the same structure as figure 4A showing a detail of the internal core;
Figure 5A shows a sketch of a core-sheath structure wherein the sheath is made from knitted weave with interlaced loop while the core is made using a braiding machine with 320 den raw silk yarn;
Figure 5B shows the same structure as figure 5A showing a detail of the internal core;
Figure 6A shows a sketch of a variant of the core-sheath structure of figure 5A wherein the outer sheath has greater beating, i.e. the loops are closer, making the overall structure slightly more compact, and consequently slightly more rigid, and with reduced porosity ("beaten" structure).
Figure 6B shows the same structure as figure 6A showing a detail of the internal core;
Figure 7A shows SEM images of cells (L929, immortalised murine fibroblasts) adhering to the textile structures of the invention 2 days after seeding;
Figure 7B shows SEM images of cells (L929, immortalised murine fibroblasts) adhering to the textile structures of the invention 7 days after seeding;
Figure 8 shows an example load/elongation curve typical of the silk structure forming the subject of the invention;
Figure 9 shows sketches simulating 6x magnification stereomicroscopic images of a structure made from 80 den yarn where both the outer sheath and the inner core are weave fabric with interlaced loops: (A) untreated overall structure; (B) degummed overall structure; (C) untreated inner core; (D) degummed inner core;
Figure 10 shows sketches simulating 6x magnification stereomicroscopic images of a structure made from 80 den yarn where the outer sheath is woven fabric with interlaced loops while the inner core is interwoven: (A) untreated overall structure; (B) degummed overall structure; (C) untreated inner core; (D) degummed inner core;
Figure 11 shows sketches simulating 6x magnification stereomicroscopic images of a structure made from 320 den yarn where the outer sheath is woven fabric while the inner core is interwoven: (A) untreated overall structure; (B) degummed overall structure; (C) untreated inner core; (D) degummed inner core;
Figure 12 shows sketches simulating 6x magnification stereomicroscopic images of a structure made from 320 den yarn where the outer sheath is woven fabric with interlaced loops while the inner core is interwoven ("beaten" structure): (A) untreated overall structure; (B) degummed overall structure; (C) untreated inner core; (D) degummed inner core;
Figure 13A shows SEM images (500x magnification) of cells (L929, immortalised murine fibroblasts) adhering to the textile structures of the invention, coated with type A gelatine, 24 hours after seeding;
Figure 13B shows SEM images (1000x magnification) of cells (L929, immortalised murine fibroblasts) adhering to the textile structures of the invention, coated with type A gelatine, 24 hours after seeding.

Advantageously, the structure of the invention consists of silk obtained from silkworms, for example, from *Bombyx mori* and related species, silk from spiders, for example *Nephila clavipes,* silk from genetically modified bacteria, yeast cells, insect cells, transgenic plants and animals.

The weave structure of the invention consists of two concentric tubular structures, an outer structure named the sheath and an inner structure named the core. The sheath is a braided weave with eyelet, while the core is a braided weave with eyelet or made using a braiding machine.

The structure is obtained by interweaving a silk yarn using a braiding machine with between 10 to 20 spindles, preferably 16 spindles, so as to obtain the inner core. The spindles consist of between 6 and 10 cones, preferably 8, and between 14 and 18 spools, preferably 16. The inner core may be made using a weaving loom with interlaced loops or using a braiding machine.

Advantageously, the braided inner core is overhauled to check the regularity of the product and the consistency for subsequent processing.

Then, the braided core is mounted on a loom for weaving of the outer sheath over the core. A number of cones, between 4 and 8, preferably 5, with a number of needles of between 4 and 8, preferably 6, are arranged on the creel, located behind the loom. Thanks to the high number of cones on the creel of the loom, it is possible to obtain a product with suitable thickness and with sufficiently large weave to allow cell colonisation inside the silk structure. At the same time, this way, it is possible to obtain a product with suitable thickness and softness.

In one variant of the process, it is possible to weave the sheath with greater beating, i.e. with closer loops so as to make the overall structure slightly more compact and consequently slightly more rigid, and with reduced porosity ("beaten" structure).

The loom used to weave the outer sheath is preferably the Semel loom.

The silk yarn used to obtain the weave structure has a linear density comprised of between approx. 40 den and approx. 640 den, preferably approx. 80 den or approx. 320 den. Denier is the unit of measurement for the linear density of a yarn. The unit "den" corresponds to 1 gram per 9000 m of yarn.

The silk yarn consists of between 2 and 8 ply of raw silk, preferably 4, twisted together with between 100 and 300 turns/metre, preferably approx. 180 turns/metre, and each ply of raw silk is made starting from 5 to 15 silk filaments, preferably 10.

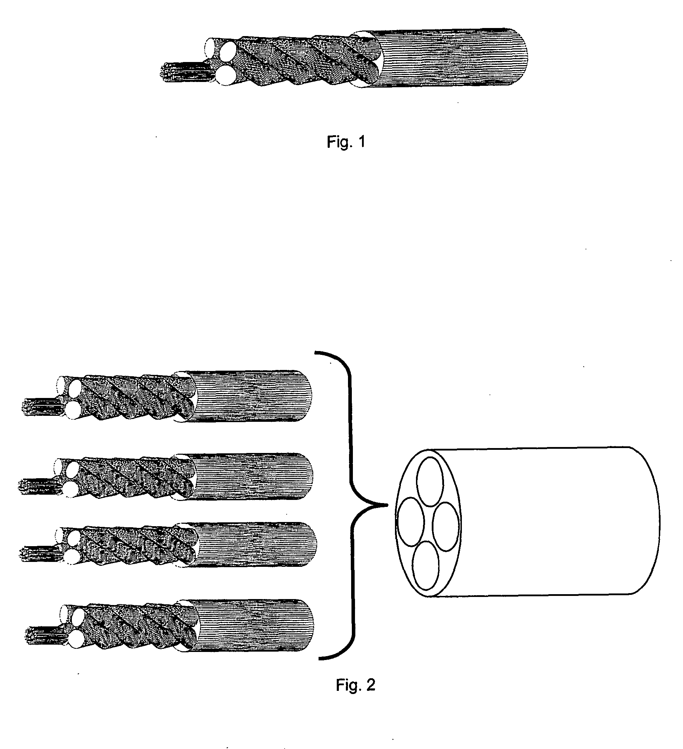

One raw silk fibre consists of two filaments of fibroin coated with sericin, hence, each ply consists of 20 fibroin filaments. The linear density of the yarn thus composed is approx. 80 denier (den) (Figure 1).

In one preferred embodiment, at least four yarns of linear density of approx. 80 den (of the type described above) are twisted together to give a yarn of linear density of approx. 320 denier (Figure 2). The yarn of approx. 320 den is obtained by assembling at least four cones of approx. 80 den yarn on a doubling machine. The four approx. 80 den yarns are then paired and twisted to give the 320 den yarn.

It has been shown experimentally that 320 den yarn gives the core-sheath structure such mechanical properties as to make the structure more suited to certain applications, such as for example, the production of a prosthetic ACL.

Both the approx. 80 den yarn and the approx. 320 den yarn may be used as starting yarns to obtain the core-sheath structure of the invention.

On completion of the weaving process, the core-sheath structure of the invention is subjected to degumming to eliminate the sericin.

The biomimetic prosthetics forming the subject of this invention preferably undergo degumming in an autoclave.

Samples for degumming are immersed in distilled water and heated to a temperature of between 105°C and 160°C, preferably 120°C. This is then followed by an isothermal phase lasting 20-60 minutes, preferably 40 minutes, where the actual degumming occurs, and a final cooling phase, which ends on reaching temperatures of less than 80°C.

On completion of cooling, the samples are removed from the autoclave and rinsed three/four times with water at approx. 40°C, in order to remove residual sericin from the fibroin fibres.

Following the degumming process, the components of the textile structure forming the subject of the invention, have a diameter of between 0.5 and 12.0 mm, preferably between 2.0 and 8.0 mm, for the core, and between 2.0 and 15.0 mm, preferably between 4.0 and 12.0 mm, for the sheath.

By application of the above-described method for preparing the core-sheath structure of the invention, an inner core is obtained with a mean pore area comprised of between 0.001 and 2.000 mm², preferably between 0.010 and 0.500 mm², and an outer sheath with a mean pore area ranging between 0.005 and 15.000 mm², preferably between 0.050 and 5.000 mm².

Both components of the textile structure have high "weave porosity" which is adequate for allowing cell colonisation, proliferation and migration at each point on the biomimetic prosthesis; furthermore, the inner zone of the biomimetic prosthesis represents a preferential zone, protected against wear phenomena, for the formation of novel ligament tissue.

Indeed, the different core and sheath morphologies have been designed to perform different tasks and functions with the aim of optimising ligament tissue regeneration.

The core, with the fibres arranged in a highly oriented manner, has the role of both guaranteeing the mechanical characteristics necessary for sustaining the loads, and providing a preferential direction for the formation of collagen fibres in the newly formed ligament.

The highly porous sheath has the role of protecting the core against wear due to rubbing as a result of the contact between the structure and the other tissues present in the joints (for example in the bone areas) at the same time allowing cell adhesion, proliferation and migration towards the interior of the biomimetic prosthesis.

The core-sheath structures obtained have peak load values comprised of between 500 and 5000 N, preferably between 1000 and 2500 N, and peak elongation values between 10% and 60%, preferably between 30% and 40%.

Mechanical stress tests performed on the textile structures have shown peak load values compatible with a double bundle type reconstruction approach, where ACL reconstruction is implemented by inserting two lengths of biomimetic prosthetic implant in the joint, thus doubling the mechanical characteristics.

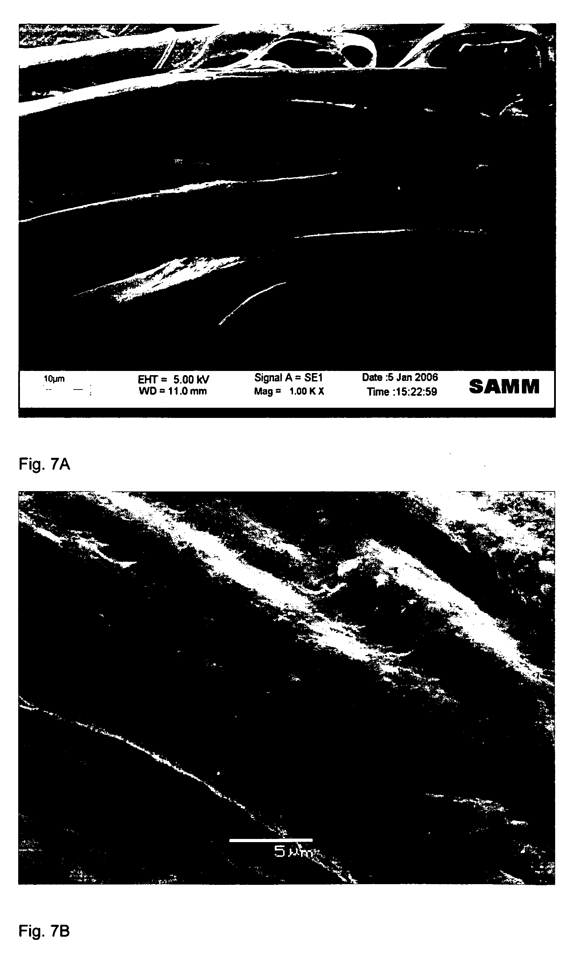

*In vitro* cell tests have confirmed the cytocompatibility of the silk fibroin used in making the textile structures. As shown in Figure 7A, the seeded cells, immortalised murine fibroblasts (L929), are capable of adhering to individual fibroin fibres after just 2 days, even penetrating inside the structure and the individual yarn. At 7 days, the cells form a uniform cell layer, completely coating the yarn (Figure 7B). The cells which have been shown to be capable of adhesion and proliferation once seeded onto the core-sheath structure of the invention *in vitro,* are fibroblasts and primary muscle cells obtained from pig aorta.

Thanks to the high porosity of the textile matrix, the biomimetic prosthetics of the invention allow cells to colonise inside the structure, at the same time guaranteeing greater available surface for the cells themselves in order to promote adhesion and proliferation. Furthermore, in addition to possessing the above-described ideal porosity characteristics, the biomimetic prosthetics of the invention also have good peak load and peak elongation mechanical properties, entirely comparable to those of natural ligaments and tendons.

Therefore, the core-sheath structures forming the subject of the invention have shown they possess the fundamental characteristics in order to be able to be an ideal support for inducing cell adhesion and proliferation and, then, the formation of novel tissue both on the surface and inside the structure.

Hence, the core-sheath structures of the invention are advantageously used as biomimetic prosthetics to be implanted in vivo for the regeneration of ligaments and tendons, particularly the anterior cruciate ligament, the posterior cruciate ligament, the collateral knee ligaments, the Achilles tendon, the elbow ligaments, the flexor tendon of the hand, the side ligaments of the ankle and the tendons and ligaments of the jaw. Other tissues that can be regenerated thanks to implantation of the structure of the invention include other tendon and ligament tissues, muscle tissues and blood vessels.

The textile structures forming the subject of the invention may be subjected to chemical treatments. Examples of chemical treatments that can be applied to the textile structures are reported in the following publications: J. Biomed. Mater. Res. 2001 Jan;54(1):139-48, Biomacromolecules. 2007 Apr;8(4):1200-8, Biomaterials. 2004 Feb;25(3):377-83, Journal of Applied Polymer Science. 2001 Apr;80 (2), 297-303) and/or physical treatments (such as for example those described in J. Zhejiang. Univ. Sci. 2004 Aug;5(8):918-22, Biomacromolecules. 2007 Nov;8(11):3548-56, Int. J. Mol. Med. 2006 Aug;18(2):241-7) and/or enzyme treatments (such as for example those described in J. Biotechnol. 2005 Mar 2;116(1):21-33, J. Biotechnol. 2006 Sep 1;125(2):281-94). The scope of such treatments is to optimise the biological, physiological and mechanical interaction characteristics of the structures of the invention with the surrounding environment in both in vivo and *in vitro* applications; indeed, by means of such treatments, it is possible to functionalise the fibroin fibres with bioactive molecules (such as for example growth factors, enzymes, drugs), molecules and/or ions and/or particles with antibacterial properties (such as for example silver ions, titanium dioxide nanoparticles), modify the chemical/structural properties of the fibroin (for example increase the degree of crystallinity of the fibroin), alter the physical and/or mechanical properties (for example, reduce the resistance to enzyme attack, with consequent increase in biodegradation rate).

In one preferred embodiment of the invention, the core-sheath structures are coated, with the purpose of improving and promoting cell adhesion and/or replacing the sericin functions, with biodegradable polymers selected from synthetic polymers and copolymers (such as for example poloxamers, biodegradable polyesters, biodegradable polyurethanes) and natural polymers (such as for example fibrin glue, fibrinogen, collagen, gelatine, alginate, chitosan, hyaluronic acid and derivatives thereof).

The core-sheath structures of the invention may be coated by means of known techniques including, for example, dipping, solvent casting, spray or electro-spray deposition.

In one preferred embodiment of the invention, the above-described coatings may be functionalised and/or supplemented with bioactive molecules (such as for example growth factors, enzymes, drugs), molecules and/or ions and/or particles with antibacterial properties (such as for example silver ions, titanium dioxide nanoparticles), autologous cells, pluripotent cells, totipotent cells.

The known methods for functionalising and/or supplementing said coatings envisage for example, simple diffusion, immobilisation by means of electrostatic interactions or covalent bonds, the use of micro- and nano- capsules (examples of such techniques are reported in the following publications: Adv. Drug Deliv. Rev. 2008 Jan. 14;60(2):229-42, Adv. Drug Deliv. Rev. 2007 May 30;59(4-5):207-33, Adv. Drug Deliv. Rev. 2007 May 30;59(4-5):187-206, Curr. Diab. Rep. 2007 Aug;7(4):314-20, Nanomedicine. 2006 Mar;2(1):8-21)

One additional application of the core-sheath structures forming the subject of the invention may concern the use of the same for the *in vitro* generation of "pretissues", i.e. partially regenerated tissues that can then be implanted *in vivo*, replacing the injured tissue. The core-sheath structures have displayed suitable characteristics for the *in vitro* regeneration of ligament and/or tendon and/or muscle and/or blood vessel tissues.

### EXPERIMENTAL EXAMPLES

### EXAMPLE 1 - TEXTILE STRUCTURES OBTAINED BY WEAVING 80 DEN OR 320 DEN RAW SILK YARNS

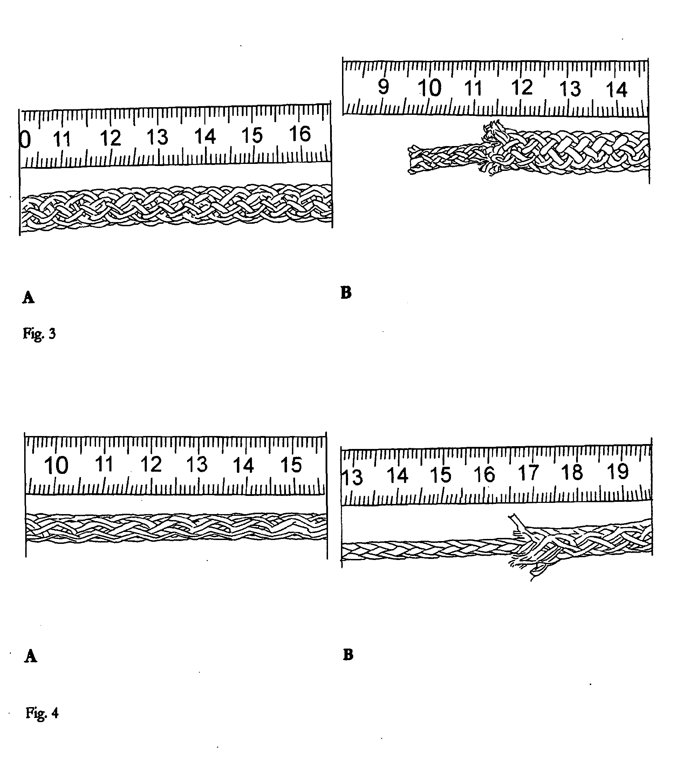

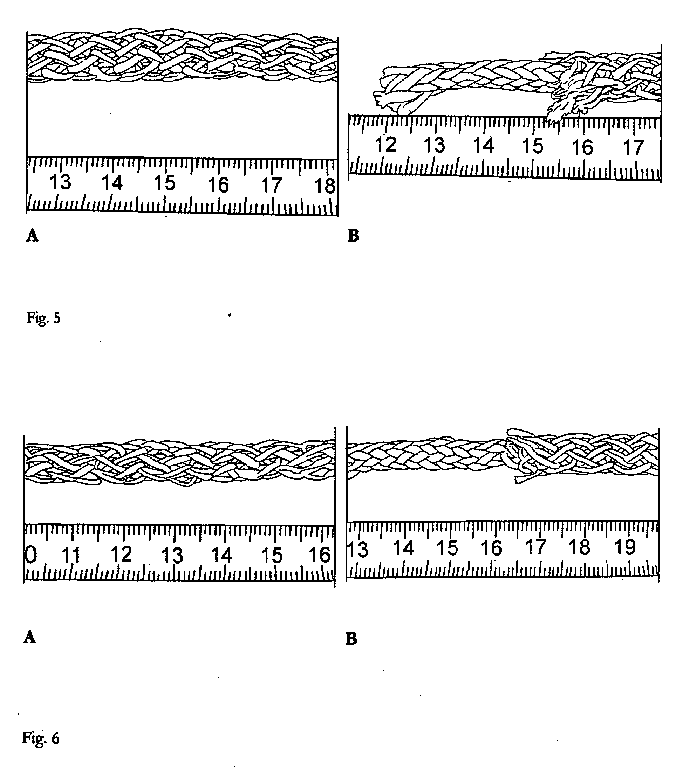

Using raw silk yarn with linear density equal to 80 den (F80) and 320 den (F320) (Figure 1 and Figure 2) textile structures consisting of two concentric tubular components have been produced, one known as the sheath (outer component) and one known as the core (inner component). The structures made using F80 yarn are labelled SS80 wherein both the sheath and the core are woven fabrics with interlaced loops (Figure 3) and TS80 where the core is a braided structure and the sheath is woven fabric with interlaced loops (Figure 4), while those obtained starting from F320 yarn are labelled TS320-A (Figure 5) consisting of a sheath of woven fabric with interlaced loops and a braided core and TS320-B (Figure 6) which differs from TS320-A due to the different sheath production process, making the overall structure slightly more compact and consequently slightly more rigid and with reduced porosity.

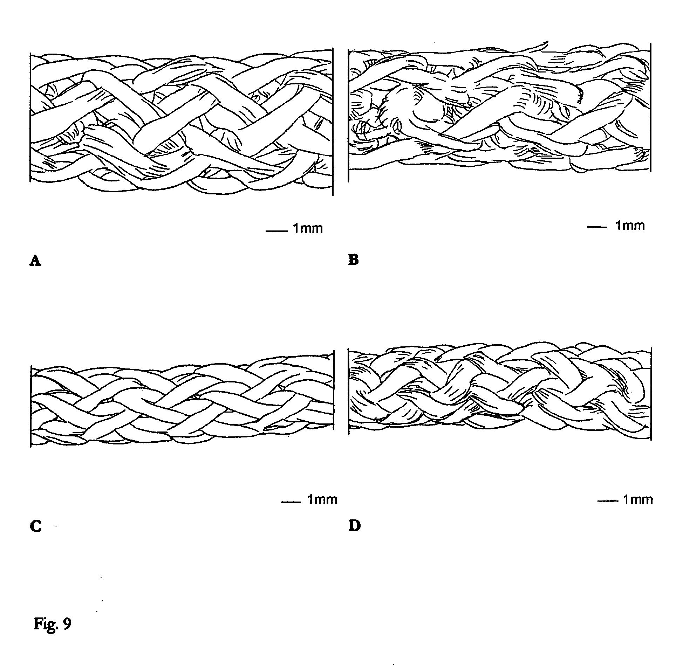

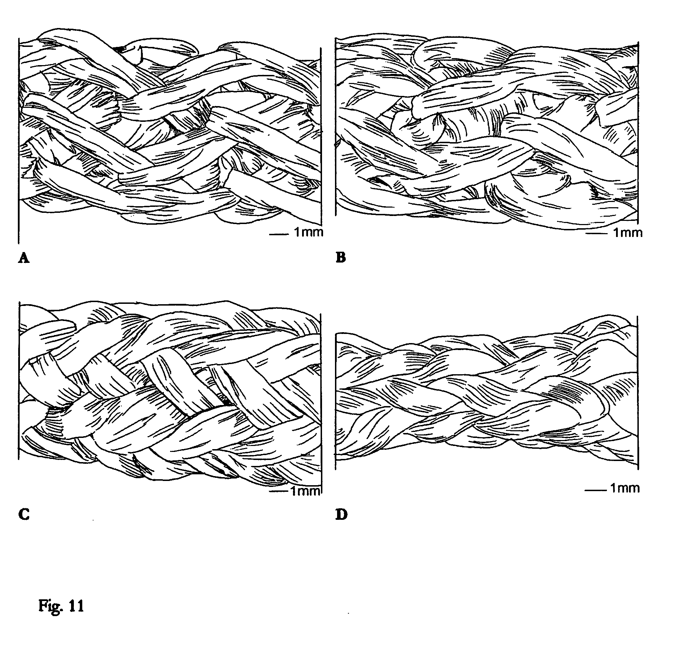

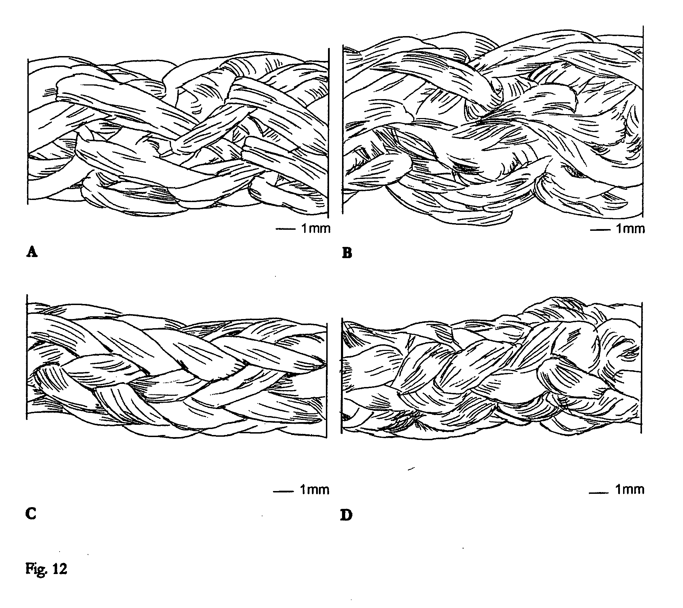

Following weaving, the structures have been subjected to degumming in an autoclave in order to remove the sericin (Figure 9, Figure 10, Figure 11, Figure 12). The degumming treatment envisages the immersion of the structures in distilled water with a wetting ratio equal to 40 ml for every gram of raw silk, and subsequent treatment in an autoclave at 120°C for 40 minutes (excluding heating and cooling times). On completion of the processing cycle, the autoclave is left to cool to 80°C and then the samples rinsed manually with tepid water (approx. 40°C). The rinsing treatment must be performed three/four times.

Table 2 reports the core and outer sheath diameters of the woven structures, in both raw and degummed forms. It may be observed that, in the raw state, the samples have greater mean diameter values, for both the inner core and the outer sheath, compared to those measured following the degumming process.

**Table 2 - Diameters of both component parts of the SS80 and TS80 structures, in both raw and degummed forms.**

| | | **Inner core diameter [mm]** | **Outer sheath diameter [mm]** |
|---|---|---|---|
| **SS80** | **Raw** | 2.55±0.05 | 6.30±0.30 |
| | **Degummed** | 2.20±0.06 | 6.00±0.40 |
| **TS80** | **Raw** | 2.12±0.04 | 5.40±0.45 |
| | **Degummed** | 1.96±0.05 | 5.10±0.50 |
| **TS320-A** | **Raw** | 3.85±0.30 | 8.80±0.70 |
| | **Degummed** | 3.60±0.45 | 8.40±0.50 |
| **TS320-B** | **Raw** | 4.15±0.20 | 8.40±0.5 |
| | **Degummed** | 3.90±0.35 | 8.00±0.7 |

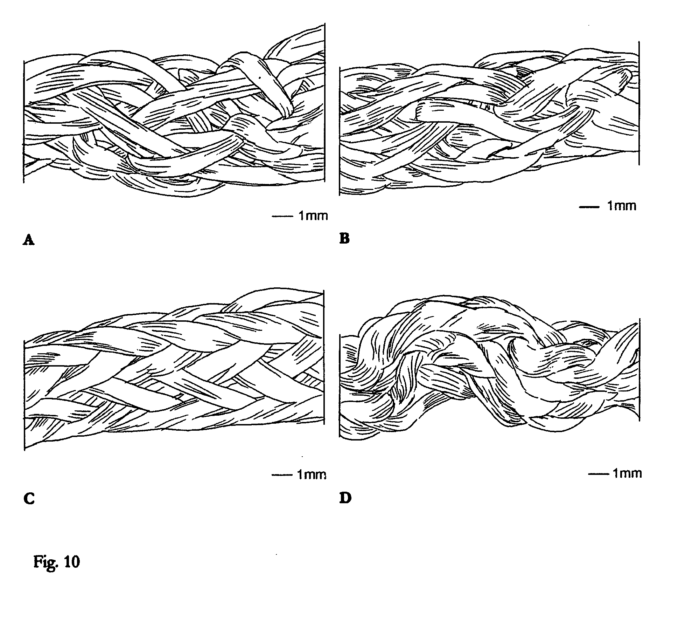

The morphology of the structures forming the subject of the invention has been observed by stereoscopic light microscopy. Both the inner core and the outer sheath, before and after the structures have been subjected to the degumming process, have been analysed for each sample. Furthermore, images have been acquired (6x magnification) of each component of the structure, in both raw and degummed forms. The images in Figure 9, Figure 10, Figure 11, Figure 12, show the morphology of the structure before (images A and C in each figure) and after (images B and D in each figure) degumming. From examination of the images shown in Figure 9 it may be observed how the sheath and the core of structure SS80 have been woven according to the same type of weave. On the other hand, examination of the images in Figure 10, Figure 11 and Figure 12 shows the different morphology of the core in the TS structures.

Using the structural images acquired by stereoscopic microscopy, with the use of AutoCAD® software, it has been possible to calculate the mean surface areas of the pores present on both the core and the sheath of the structure. Closed lines (polylines) matching the outlines of the void areas in the structures have been traced using the program's "polyline" function. The polylines are identified by the software as geometric entities for which the boundary length and the surface extent may be calculated. The area value for a pore, expressed in mm², has then been deduced by normalising the area measurement provided by the software compared to the area of the marker present in the surface image, known to be equal to 1 mm²_{.}

Table 3 reports the mean values and the corresponding standard deviations of the pore areas identifiable on the surface of the inner core and the outer sheath of the structure, before and after degumming .

**Table 3 - Mean area of the pores present on the inner core and on the outer sheath of the raw and degummed structures.**

| **Structure** | **Component** | **Mean pore area [mm²]** | |
|---|---|---|---|
| | | **Raw** | **Degummed** |
| **SS80** | **Core** | 0.077±0.019 | 0.022±0.009 |
| | **Sheath** | 1.120±0.270 | 2.090±0.140 |
| **TS80** | **Core** | 0.098±0.006 | 0.044±0.011 |
| | **Sheath** | 1.520±0.140 | 2.550±0.340 |
| **TS320-A** | **Core** | 0.160±0.014 | 0.110±0.034 |
| | **Sheath** | 3.860±0.090 | 2.520±0.080 |
| **TS320-B** | **Core** | 0.048±0.007 | 0.070±0.016 |
| | **Sheath** | 3.980±0.210 | 2.370±0.790 |

Considering the value of the mean area of the pores present on the surfaces of the inner cores of structures SS80, TS80 and TS320-A, a reduction in surface area is observed as a result of the degumming process, which causes shrinkage of the structure. The inner core of structure TS320-B does not undergo this contraction, and a greater mean pore area value is measured for the degummed structure compared to those in the raw form.

The outer sheaths are characterised by geometries that are less compact compared to the inner cores; indeed, as may be seen from the values reported in Table 3, the dimensions of the pores present on the outer part are almost two orders of magnitude greater compared to the areas of the pores present on the core.

By analysing the changes induced by the degumming process on the outer sheathes of the structures under investigation, a different morphology is observed depending on the yarn used for weaving the structure. For the samples obtained starting from F80 yarn, an increase in pore surface area may be observed following removal of the sericin. On the other hand, the sheath of TS320-A and TS320-B structures undergoes shrinkage of the pores caused by the degumming process.

Mechanical characterisation of the degummed structures has been performed according to the method described in the document issued in 1997 by the FDA (U.S. Food and Drug Administration, "*Guidance document for the preparation of investigational device exemptions and premarket approval applications for intra-articular knee ligament device";* 1987 (Revised 1993, Reformatted 1997)).

In an unpublished paper (A. Alessandrino: "silk textile structures as scaffolds for engineering the anterior cruciate ligament"; Degree Thesis submitted to Milan Polytechnic, academic year 2003/2004), it has been verified that the rate of deformation does not influence the behaviour of silk textile structures and, hence, it has been decided to conduct the tests solely at a rate of deformation equal to 2%/s, using test pieces with usable length equal to 30 mm. Uniaxial tension tests have been conducted at the Biomaterials Laboratory, Department of Bioengineering, Milan Polytechnic, using an MTS 1/MH tension test machine and pneumatic grips with a gripping pressure of 4 bar and a preload of 1 N.

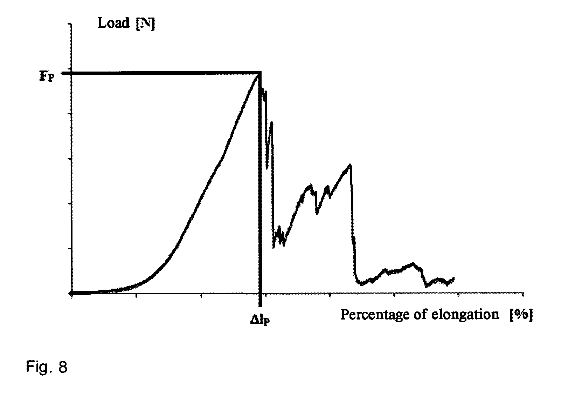

Figure 8 shows an example load/elongation curve characteristic of the silk structures, where the parameters under consideration may be identified, such as:
- Fp: the peak load value, expressed in Newtons [N];
- Δlp: the elongation at peak load, expressed as a percentage [%].

The values of the mechanical parameters, derived from the load/elongation curves for the various degummed structures, are reported in Table 4.

**Table 4 - Values of the mechanical parameters obtained from the tension tests.**

| | **Peak load IN]** | **Elongation at peak [%]** |
|---|---|---|
| **SS80** | 430±60 | 55,2±7,8 |
| **TS80** | 942±65 | 37,2±9,9 |
| **TS320-A** | 1531±75 | 32,1±2,6 |
| **TS320D-B** | 1477±110 | 35,4±3,7 |

In the initial part of the load/elongation curves (not shown) a section with an exponential trend, where a significant increase in load with increasing deformation is not seen, has been observed for all the structures. This zone, corresponding to the recovery of the deformation induced on the fibres by the degumming process causing the structure to shrink, is more marked for the SS80 structure, while it is less extensive for the TS80, TS320-A and TS320-B structures. Once the fibres are realigned, the curve follows a linear trend where a rapid increase in load is observed until reaching the peak. The load/elongation curves of the SS80 and TS80 structures are characterised by the presence of secondary peaks, both before and after the main rupture peak, corresponding to the partial and progressive breakdown of the yarn. On the other hand, the trend of the load/elongation curves of the TS320-A-S and TS320-B-S structures is characterised by the absence of secondary peaks both before and after the peak corresponding to maximum load. Furthermore, the abruptly descending section highlights the abrupt rupture of the test piece.

The structures examined in this experimental example may be used for the regeneration/replacement of any ligament and/or tendon and/or muscle tissue where the chemical-physical-mechanical characteristics are suitable.

### EXAMPLE 2 - COATING OF TEXTILE STRUCTURES OBTAINED BY WEAVING A 320 DEN RAW SILK YARN WITH POLOXAMER 407

The polymer gel coating of textile matrices is a method that can be used to optimise both *in vitro* cell interaction, and in vivo biological response. Indeed, chemotactic factors, growth factors, or even cells can be dispersed inside the coating. Furthermore, the coating can allow mimicking of mechanical functions performed by sericin in raw silk.

The presence of a polymer coating gives the textile structures a different response to mechanical stress, influencing the spatial reorganisation abilities of the fibres. The testing conducted using poloxamer 407 gel as coating material is reported by way of example.

The poloxamers represent a group of biodegradable polymers used extensively in the pharmaceutical industry, mostly in the gel state, as carriers for drug delivery. In this experimental section, poloxamer 407, identified by the brand name Lutrol® F127 (BASF), has been considered. The peculiar characteristic of such gels is their thermoreversibility; particularly, the viscosity depends on the concentration of the solution and the temperature at which the material finds itself, displaying increasing viscosity with increasing temperature. The viscosity of an aqueous solution of poloxamer 407 follows a trend depending on the temperature and the concentration of the solution. Poloxamer 407 gel shows maximum viscosity at temperatures between 30°C and 60°C and liquid characteristics for temperatures close to 0°C or greater than 80°C. Another peculiar characteristic of this material is that it is rapidly degradable in aqueous environments.

Poloxamer gel has been produced starting from solution of poloxamer 407 (BASF) grains in distilled water at a concentration of 25% w/v. Since this is a thermo-reversible material with rheological properties that vary depending on the temperature, once the concentration of the solution is fixed, the polymer dissolves completely in water within a temperature range of between 0°C and 5°C, where the material is in the liquid state. After having obtained a homogeneous solution of poloxamer, this has been inserted in a 5 ml hypodermic syringe and has then been injected inside the TS320-A and TS320-B structures, previously described in EXAMPLE 1, obtaining a uniform distribution of the gel. The structures thus prepared have then been conditioned at room temperature for 24 hours in order to allow the correct solidification of the poloxamer 407 gel.

Mechanical characterisation of the composite structures thus obtained has been performed according to the methods described in EXAMPLE 1 and has given the results shown in Table 5.

**Table 5 - Values of the mechanical parameters obtained from the tension tests conducted on the TS320-A composite and TS320-B composite structures.**

| | **Peak load [N]** | **Elongation at peak [%]** |
|---|---|---|
| **TS320-A composite** | 964±31 | 59,4±13,5 |
| **TS320-B composite** | 1085±93 | 57,9±9,4 |

The presence of the polymer gel coating influences the ability of the textile matrix fibres to reorganise, reducing the ability of the fibres to align themselves in the direction of load application. Evidence of this, from the load/elongation curves (not shown), includes the absence of a first section where there is spatial reorganisation due to the low load values applied and the reduced rupture load values of the composite structures, if compared with textile structures with no polymer coating.

The poloxamer 407 coating studied here is reported by way of example; other materials such as biodegradable polymers selected from synthetic polymers and copolymers (such as for example poloxamers, biodegradable polyesters, biodegradable polyurethanes) and natural polymers (such as for example fibrin glue, fibrinogen, collagen, gelatine, alginate, chitosan, hyaluronic acid and derivatives thereof), may be similarly used to make said coating.

### EXAMPLE 3 - ANALYSIS OF THE IN VITRO INTERACTION OF THE TEXTILE STRUCTURES WITH L929 CELLS

With the aim of optimising the cell interactions of the textile structures and in order to facilitate cell adhesion, growth and proliferation in each area of the textile structures forming the subject of the invention, an *in vitro* experiment has been performed with L929 cells, coating the textile structures with type-A gelatine (SIGMA-ALDRICH).

Aqueous solutions of gelatine have been prepared at concentrations equal to 1% w/v and 10% w/v, kept at 37°C and sterilised by filtration. Type TS80 textile structures, described in EXAMPLE 1, have been degummed according to the method described in EXAMPLE 1 itself, sterilised by treatment at 121°C in an autoclave for 15 minutes, and then subsequently immersed in the various gelatine solutions for 30 minutes.

Murine fibroblasts from the L929 cell line (ECACC, European Collection of Cell Culture) have been seeded on the composite structures obtained, and incubated for 24 hours in an incubator at 37°C and 5% CO₂. After incubation for 24 hours, the samples have been prepared for observation by Scanning Electron Microscope by means of an initial treatment in glutaraldehyde and subsequent treatments in ethanol-water solutions at increasing ethanol concentrations.

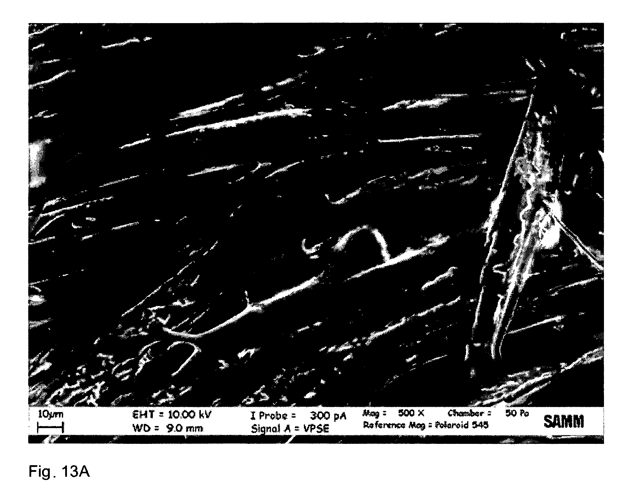

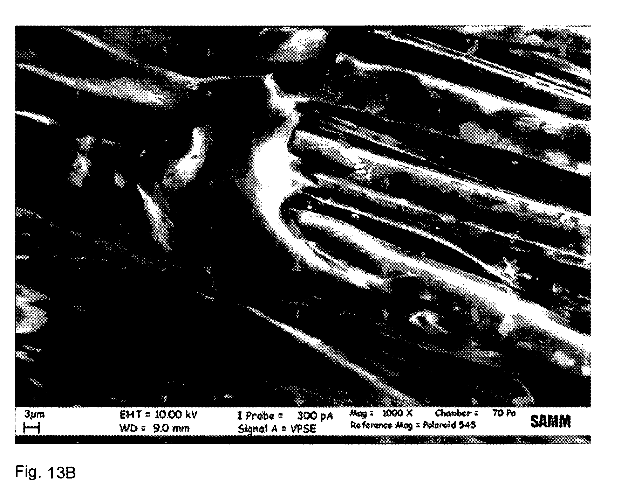

As may be observed from the images reported in figures 13A and 13B, the textile structures promote the adhesion of L929 cells to the fibroin fibres of the outer sheath and the inner core.

## Claims

1. A silk fibroin weave structure comprising a concentric tubular outer sheath and an inner core, wherein said sheath is a woven fabric with interlaced loops and said core is a woven fabric with interlaced loops or is braided.

2. The structure according to claim 1, wherein said silk fibroin is a silk yarn with a linear density of between approx. 40 den and approx. 640 den, preferably, the linear density is approx. 80 den or approx. 320 den.

3. The structure according to any claim 1 to 2, wherein said core has a mean pore area of between 0.001 and 2.000 mm², preferably between 0.010 and 0.500 mm².

4. The structure according to any claim 1 to 3, wherein said outer sheath has a mean pore area of between 0.005 and 15,000 mm², preferably between 0.050 and 5,000 mm²_{.}

5. The structure according to any claim 1 to 4, wherein said core has a diameter of between 0.5 and 12.0 mm, preferably between 2.0 and 8.0 mm, while said sheath has a diameter of between 2 and 15 mm, preferably between 4 and 12 mm.

6. The structure according to any claim 1 to 5, having a peak load value comprised of between 500 N and 5000 N, preferably 1000 and 2500 N.

7. The structure according to any claim 1 to 6, having peak elongation values comprised of between 10% and 45%, preferably between 30% and 40%.

8. The structure according to any claim 1 to 7 wherein the fibres used are silk fibroin obtained from lepidoptera, for example from *Bombyx mori* and related species, from arachnids, for example *Nephila clavipes,* from genetically modified bacteria, yeast, insect cells and transgenic plants and animals.

9. The structure according to any claim 1 to 8 wherein the silk fibroin fibres are functionalised with bioactive molecules, by means of chemical and/or physical and/or enzyme treatments.

10. The structure according to any claim 1 to 9, wherein said structure is coated with biodegradable polymers selected from synthetic polymers and copolymers, for example poloxamers, biodegradable polyesters, biodegradable polyurethanes and natural polymers, for example fibrin glue, fibrinogen, collagen, gelatine, alginate, chitosan, hyaluronic acid and derivatives thereof.

11. The structure according to claim 10 wherein said biodegradable polymers are supplemented and/or functionalised with chemotactic factors, growth factors, enzymes, drugs, autologous cells, pluripotent cells, totipotent cells.

12. A process for the preparation of the structure according to any claim 1 to 8, comprising steps of:
a) weaving, to a fabric with interlaced loop, using a loom with 4 to 8 cones, or braiding using a braiding machine with 10 to 20 spindles, a silk yarn to give the core;
b) mounting the core on a loom;
c) weaving the outer sheath over the core using a loom with 4 to 8 cones and 4 to 8 needles.

13. The process according to claim 12, wherein, in step a), said spindles are comprised of 6 to 10 cones and 14 to 18 spools.

14. The process according to claims 12 or 13, further comprising a degumming step of said structure, in order to eliminate the sericin.

15. The structure according to any claim 1 to 11 for use in *in vivo* cell adhesion and proliferation.

16. The structure according to claim 15 for use in the *in vivo* regeneration of tissues.

17. The structure according to claim 16, wherein said tissues are selected from the group consisting of ligaments, particularly the anterior cruciate ligament, tendons, muscles and blood vessels.

18. The structure according to any claim 1 to 11 for use in *in vitro* cell adhesion and proliferation.

19. The structure according to claim 18 for use in the *in vitro* regeneration of tissues.

20. The structure according to claim 19, wherein said tissues are selected from the group consisting of ligaments, particularly the anterior cruciate ligament, tendons, muscles and blood vessels.
